# EUROPEAN PATENT SPECIFICATION

(11) **EP 0 621 015 B1**
(45) Date of publication and mention of the grant of the patent: **18.03.1998**
(21) Application number: 93106646.8
(22) Date of filing: 23.04.1993
(51) Int. Cl.: A61F 2/06

(54) **Stent with a covering layer of elastic material and method for applying the layer on the stent**
Stent mit einer Beschichtung aus elastischem Material und Verfahren zum Anbringen der Beschichtung auf dem Stent
Endoprothèse avec une couche de couverture en matériau élastique et procédé pour appliquer la couche sur l'endoprothèse

(43) Date of publication of application: 26.10.1994
(73) Proprietor: SCHNEIDER (EUROPE) AG, 8180 Bülach (CH)
(72) Inventor: Lukic, Goran, CH - 8180 Bülach (CH)
(74) Representative: Misrachi, Alfred

(56) References cited:
- EP-A- 0 430 542
- EP-A- 0 435 518
- EP-A- 0 481 365
- DE-A- 3 918 736
- DE-A- 4 022 956
- GB-A- 1 205 743
- US-A- 3 738 365
- US-A- 3 879 516
- US-A- 5 180 376

## Description

This invention relates to a stent as specified in the preamble of Claim 1. Such a stent is known e.g. from DE-A-3918736. This invention also relates to a method for applying such a layer on a stent.

The discontinuous walls of stents, such as for instance the macroporous walls formed by a deformable wire mesh allowing diametral retraction for introduction of the stent into air or food pipes and expansion therein for dilatation, or repair, or bridging of said pipes, have the disadvantage that they permit ingrowth of tumors and other rapid growth cells through the wire mesh or discontinuous wall, with the resulting risk of stent occlusion.

For preventing ingrowth of a tumor through the stent, the document EP-A-0481365 shows an endoprosthesis made of a temperature controlled self-expandable metallic wire structure which can be embedded in a silicon material which results in a zig-zag or undulated pattern that closely follows the pits and bosses forming the relief of the lattice structure. Such a coating configuration does not have enough physical strength in the pits of the structure to prevent tumor cells ingrowth through the stent. Furthermore, in case of degradation of the coating inside the stent due, i.a., to cell ingrowth, there is a risk of having flaps and the like resulting from the damaged coating which could occlude the vessel or migrate therethrough and cause an occlusion at some other place.

Also to prevent ingrowth of unwanted cells into the stent, the document DE-A-3918736 describes an expandable metallic stent with either a dispersion of PTFE on the wire mesh, or an inner PTFE tube heat adhered or sewn to the stent, or inner and outer PTFE tubes adhered to one another between the bosses of the wire mesh or sewn pouch like to one another. The dispersion of PTFE is not resilient and will result in an embedding of the wire mesh, perhaps in a coating taking a zig-zag pattern following the pits and bosses forming the relief of the lattice structure, with the same disadvantages as outlined hereabove, and with the added disadvantage that the absence of flexibility of the PTFE coating or embedding may strongly raise the risk of having flaps inside the stent due to the degradation of the coating or due to the damage occurring on the coating during expansion of the stent. The first alternative of an inner PTFE tube may experience the same drawbacks and in case of a degradation of the inner tube, in addition to the risk of having flaps from the inner tube occluding the vessel there will be a risk of migration of the inner tube with respect to the stent and a further risk of occlusion of the vessel. The second alternative of inner and outer PTFE tubes will also have the drawbacks outlined hereabove resulting from the presence of the PTFE embedding or PTFE inner tube in addition with the risk of migration of the PTFE tubes with respect to the stent. And in both cases, the only way tought by the document to somewhat compensate the lack of resiliency of the PTFE structure is to make use of place consuming measures such as zig-zag folds on the PTFE tubes.

The document "Endoscopy 1992 : 416-420" also describes an expandable metallic stent for preventing ingrowth of malignant structures. This stent, formed by an expandable wire mesh, is covered by a silicone membrane or skirt which surrounds a portion of its length.

This membrane or skirt is secured around the stent by suture of its ends to the wire mesh, and, in situ, the membrane is thus radially held in place between the stent wall and vessel wall. To have the membrane or skirt positioned between the stent wall and vessel wall is advantageous in case of degradation of the membrane. However, such a coverage of the stent is far from being effortless and mostly will have to be done by hand, which will require skills. In addition, it is limited to certain types of materials and it may prove fragile, being possible to have the membrane or skirt getting loose from the wire mesh, which may allow relative movement between the membrane and the stent, with the resulting risk of occluding the vessel.

The document EP-A-0430542 describes a method for forming a flexible tube including a braid adhesively surrounded by a sheath and a flex in a form of a spirally wound metal strip inserted into the sheath.

The document US-A-3879516 describes a catheter having inner and outer layers of a soluble polymer composition whose adjoining surfaces are chemically bonded and a filament core in the form of a coil of the same polymer is embedded within and chemically bonded to one of the layers.

The document EP-A-0435518 describes a catheter, with the possibility of shaping it to form a stent, comprising a plastic sheath in which is friction fitted a coil.

The document US-A-5180376 describes an introducer sheath for percutaneous introduction of transluminal catheters, comprising a metallic helical coil covered by a plastic tube and the method of obtaining this assembly may be heat shrinking of the plastic tube over the coil. Alternatively, the method may be to dip coat the coil into a liquid plastic that hardens onto the helical coil after dipping. In both cases the plastic tube has extensions of material projecting into the space between the adjacent turns of the coil to prevent unwanted longitudinal movement between the turns of the coil when the sheath is severely bent.

The object of this invention is to avoid the aforesaid drawbacks.

To this effect, the stent and method in accordance with the invention comply with the definitions given in the claims.

In that way, the continuous covering layer is closely bound to the discontinuous structure which it covers and there is definitely no risk of separation therebetween. And even in the case of a strong degradation of the covering layer in course of time, there cannot be any migration of the covering layer with respect to the discontinuous wall of the stent because of the aforesaid intimal interconnection. Furthermore, the liaison of the covering layer with the discontinuous wall of the stent eliminates any delicate, time and skill consuming efforts and allows coating of any kind of discontinuous expandable stent wall.

The invention will now be described more particularly with reference to the accompanying drawings which show, by way of example only, one embodiment of the invention.

In the drawings :
Figure 1 is a perspective view of a quarter cut along the longitudinal axis of the exemplified embodiment;
Figure 2 is an enlarged view of an axial cut of a portion of its wall during a procedure for applying the covering layer.

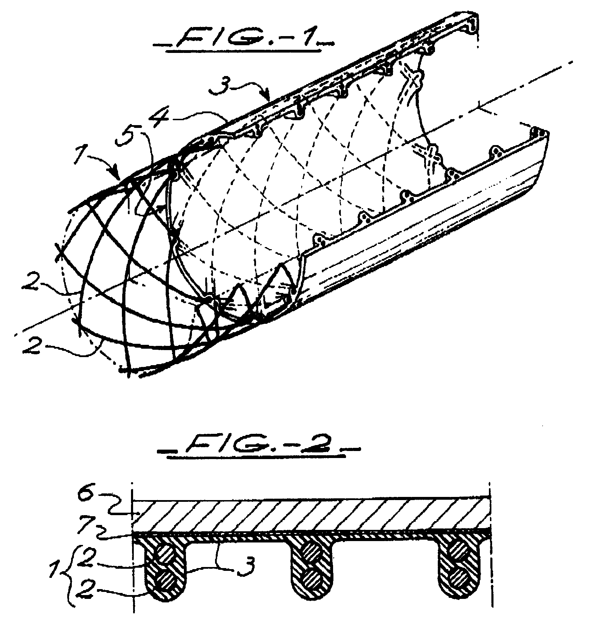

The stent shown in Figure 1 is an expandable stent of which the wall (1), for instance cylindrical, is formed by meshed wires (2) of stainless steel, plastics or hybrid materials such as plastics and carbon fiber.

The wall (1) comprises, on a portion of its length, a covering layer (3) made of an elastomeric biocompatible composition such as, for instance, the elastomeric polymerisable composition described in US Patent N° 5,112,900. The outer face (4) of layer (3) forms a surrounding surface, and layer (3) extends around and inside the discontinuous structure of the stent in order to totally embrace and intimately unite with any material part of the meshed wires (2) which constitute said discontinuous structure.

On Figure 1, the left front face (5) of the covering layer (3) is shown in an area of wall (1) where the wires (2) do not cross each other; on the contrary, the quarter cut along the longitudinal axis is shown in an area where the wires (2) cross and overlap each other.

A portion of the stent wall (1) is shown on Figure 2 with its covering layer (3), the stent wall (1) being shown in an area where its wires (2) overlap each other, and the stent being inserted in a tube (6) the inner surface of which is coated with a lifting medium (7) as described in detail hereafter in connection with a procedure for applying the covering layer to the stent.

In order to apply the covering layer (3) on the stent, the deformable wall (1) of the stent is radially contracted and the portion thereof which has to be coated is inserted into the tube (6) the inner surface of which has been previously done over with a lifting medium (7) such as for instance TEFLON (trade mark) in order to avoid adherence to the elastomeric composition forming the covering layer (3). The contracted stent is allowed to expand radially in the tube (6) and the assembly of the tube and stent is wetted with the elastomeric polymerisable composition dissolved in a sufficient amount of solvent to permit wet forming of a continuous covering layer around the totality of the discontinuous wall of the stent formed by the wire mesh inside the tube (6). The solvent is evaporated and the elastomeric composition is then polymerised in the tube and the layer covered stent portion is taken out of the tube.

In that way, the shaping and liaison of the covering layer with the discontinuous wall of the stent is obtained automatically by mass polymerisation of the elastomeric composition wholly surrounding the structure of such a wall inside the tube moulding its outer surface.

Of course, the discontinuous wall of the stent may also be covered with the continuous covering layer all over its length, in which case the stent will be fully inserted into the tube for the dip forming process. In addition, the invention is not limited to the embodiment shown, being applicable to any kind of expandable stent having a discontinuous wall.

The thickness of the covering layer may be advantageously selected as a function of the quantity of solvent added to the elastomeric composition, before polymerisation and within the limits of a fluidity sufficient to allow wetting.

As a variant, it is also possible to obtain a greater thickness of the portions of the covering layer which are located at the outside of the discontinuous wall of the stent and between the mesh or elements thereof. To this effect, the tube (6) done over with the lifting medium is first wetted alone with the elastomeric composition previously added with an appropriate amount of solvent. The solvent is evaporated and the stent is then radially contracted for insertion into the tube and the procedure follows as outlined hereinbefore.

Of course, in all these variants, the discontinuous wall of the stent may be covered with the continuous covering layer all over its length or only over a portion thereof.

## Claims

1. A stent with a discontinuous expandable wall (1) comprising on at least a portion of its length a continuous covering layer (3) of elastic material being adhered to the said portion of the discontinuous wall (1) of the stent and being thereby intimately united with said wall portion, said covering layer (3) having an outer surface (4) surrounding the discontinuous wall (1), characterized in that the continuous covering layer (3) of elastic material is shaped into an outer cylindrical surface (4) without irregularities that surrounds the discontinuous wall at the outside and extends radially within the said portion of the discontinuous wall (1) and forms an inner surface following in a distance the material parts of the discontinuous wall in places with material parts and following the cylindrical outer surface in places without material parts thereby forming an inner surface with irregularities.

2. A method for applying the covering layer of the stent according to claim 1, characterized by the steps of :
- radially contracting the stent,
- inserting at least a portion of the contracted stent into a tube the inner surface of which has been previously done over with a lifting medium,
- allowing the stent to radially expand in the tube,
- wetting the assembly tube plus stent with an elastomeric polymerisable composition dissolved in a sufficient amount of solvent to permit wet forming,
- evaporating the solvent,
- polymerising the elastomeric composition in the tube, and
- taking the layer covered portion of the stent out of the tube.

3. A method according to claim 2, wherein said tube is first wetted alone with the elastomeric composition added with solvent, and wherein the solvent is evaporated before the step of insertion of the stent into the tube.

## Patentansprüche

1. Stent mit einer unterbrochenen dehnbaren Wand (1), die auf mindestens einem Teil seiner Länge eine durchgehende bedeckende Schicht (3) aus elastischem Material umfasst, die an diesem Teil der unterbrochenen Wand (1) des Stents anhaftet und dadurch eng mit diesem Wandteil verbunden ist, und diese bedeckende Schicht (3) eine äussere Oberfläche (4) aufweist, die die unterbrochene Wand (1) umgibt, dadurch gekennzeichnet, dass die durchgehende bedeckende Schicht (3) aus elastischem Material zu einer äusseren zylindrischen Oberfläche (4) ohne Unregelmässigkeiten geformt ist, die die unterbrochene Wand auf der Aussenseite umgibt und sich innerhalb dieses Teils der unterbrochenen Wand (1) radial ausdehnt und eine innere Oberfläche bildet, an Stellen mit Materialteilen den Materialteilen der unterbrochenen Wand in einem Abstand folgt und an Stellen ohne Materialteilen der zylindrischen äusseren Oberfläche folgt und dadurch eine innere Oberfläche mit Unregelmässigkeiten bildet.

2. Verfahren zum Auftragen der bedeckenden Schicht des Stents nach Anspruch 1, gekennzeichnet durch folgende Schritte:
- radiales Zusammenziehen des Stents,
- Einführung von mindestens einem Teil des zusammengezogenen Stents in eine Rohre, deren innere Oberfläche zuvor mit einem straffenden Mittel überzogen wurde,
- Zulassen der radialen Ausdehnung des Stents in der Röhre,
- Benetzen der Verbindung von Röhre und Stent mit einer polymerisierbaren Elastomerzusammensetzung, die in einer für nasses Formen ausreichenden Menge an Lösemittel gelöst ist,
- Verdampfen des Lösemittels,
- Polymerisieren der Elastomerzusammensetzung in der Röhre, und
- Herausnehmen des schichtbedeckten Teils des Stents aus der Röhre.

3. Verfahren nach Anspruch 2, wobei diese Röhre zuerst nur mit der Elastomerzusammensetzung mit Zugabe an Lösemittel benetzt wird, und wobei das Lösemittel vor dem Schritt der Einführung des Stents in die Röhre verdampft wird.

## Revendications

1. Endoprothèse munie d'une paroi extensible discontinue (1) comprenant sur au moins une partie de sa longueur une couche de recouvrement continue (3) en un matériau élastique qui est collée sur ladite partie de la paroi discontinue (1) de l'endoprothèse et qui fait ainsi corps de façon intime avec ladite partie de paroi, ladite couche de recouvrement (3) comportant une surface externe (4) entourant la paroi discontinue (1), caractérisée en ce que la couche de recouvrement continue (3) en un matériau élastique est conformée selon une surface cylindrique externe (4) sans irrégularités qui entoure la paroi discontinue au niveau de l'extérieur et s'étend radialement à l'intérieur de ladite partie de la paroi discontinue (1) et forme une surface interne qui suit sur une certaine distance les parties de matériau de la paroi discontinue en des endroits munis de parties de matériau et qui suit la surface externe cylindrique en des endroits dépourvus de parties de matériau pour ainsi former une surface interne avec des irrégularités.

2. Procédé d'application de la couche de recouvrement de l'endoprothèse selon la revendication 1, caractérisé par les étapes de :
contraction radiale de l'endoprothèse ;
insertion d'au moins une partie de l'endoprothèse contractée à l'intérieur d'un tube dont la surface interne a reçu préalablement un milieu anti-adhésif ;
action pour faire en sorte que l'endoprothèse s'étende radialement dans le tube ;
mouillage de l'assemblage tube plus endoprothèse à l'aide d'une composition élastomérique polymérisable dissoute dans une quantité suffisante de solvant pour permettre une formation par mouillage ;
évaporation du solvant ;
polymérisation de la composition élastomérique dans le tube ; et
extraction de la partie recouverte d'une couche de l'endoprothèse hors du tube.

3. Procédé selon la revendication 2, dans lequel ledit tube est tout d'abord mouillé seul à l'aide de la composition élastomérique à laquelle est ajouté un solvant et dans lequel le solvant est évaporé avant l'étape d'insertion de l'endoprothèse à l'intérieur du tube.
